# EUROPEAN PATENT APPLICATION

(11) **EP 0 860 167 A1**
(43) Date of publication of application: **26.08.1998**
(21) Application number: 97101450.1
(22) Date of filing: 30.01.1997
(51) Int. Cl.: A61K 9/51

(54) **RNA-and DNA- based active agents in nanoparticles**

(71) Applicant: Gurny, Robert, 1211 Genève (CH)
(72) Inventor: Gurny, Robert, 1211 Genève (CH)
(74) Representative: Ryffel, Rolf

(57) **Abstract**

The invention relates to a pharmaceutical composition which is suitable for the incorporation of RNA- and DNA-based active agents in nanoparticles, which are efficient as drug carriers. The active agents may be oligonucleotides or so-called "smart biological bombs". The composition is characterized by the following components:
a) the active agent to be incorporated within nanoparticles by dispersion methods;
b) a pharmaceutically acceptable biodegradable polymer which is suitable for the formation of nanoparticles;
c) and, optionally, further pharmaceutically acceptable additives.

## Description

### Introduction

The present invention relates to a pharmaceutical composition which is suitable for encapsulating or embedding RNA- and DNA-based active agents in nanoparticles, to a process for the preparation of said composition and to the use thereof in a therapeutic method for treating the human or animal body.

### Background of the Invention

Subsequent to its enteral or parenteral administration, a therapeutic agent is distributed within the body as a function of its physico-chemical properties and molecular structure. In general, the effective amount of a drug reaching its target site is only a small fraction of the dose administered. Moreover, accumulation at other than the target sites may generate adverse reactions and undesirable side effects.

Particularly preferred target sites for therapy are endocytosing cells, especially present in the reticulo-endothelial system (liver, spleen, bone marrow), or any cells of the cellular immune system, for example the cells of the monocytic system such as blood monocytes or alveolar or peritoneal macrophages. Endocytosis by monocytes is a known immune response in the "defence" against infections, especially virus infections, or against primary tumor cells occurring in tissue as well as in blood and lymph, but also against metastases.

To effect endocytosis, the incorporation of the therapeutic agent in submicroscopic polymeric particles has been proposed. In these dispersions the active agent is encapsulated or preferably embedded in particles having a particle size smaller than 1 µm. The "loaded" particles form with the aqueous carrier liquid an aqueous phase of colloidally dispersed or, preferably, finely dispersed character. These particles are then "recognized" as suitable objectsby the cells effecting endocytosis, e.g. macrophages. Endocytosis of lipid or polymer particles containing the therapeutic agent will then further stimulate an immune response of the macrophages against antigens.

Various types of disperse systems based on lipid or polymer particles for delivering therapeutic agents have been suggested, such as micells, mixed micells, reversed micells, or unilamellar or multilamellar liposomes. Their use in therapy has gained only limited acceptance due to various problems such as instability of the carrier to be administered, leakage of the therapeutic agent from the systems, or poor storage stability. In general, such systems have proved to be inadequately effective in delivering efficacious amounts of the therapeutic agent to the target site.

The problem, to which the present invention relates, is defined as follows: Therapeutic agents of macromolecular structure, such as RNA- and DNA-based active agents, are to be delivered to their specific target site incorporated in small particles to effect endocytosis. In *French Published Patent Application No. 2 724 935*, nanoparticle dispersions are suggested containing therapeutic agents of the nucleic acid type as well as fragments or derivatives thereof. Although this reference alleges that the nucleic acid matter is enclosed or encapsulated within nanoparticles, the amount of therapeutic agent present in the nanoparticles remains low. This can be explained by the method of preparing the nanoparticles for drug delivery disclosed in this reference. The nanoparticles are loaded with the nucleic acid matter by an incubation method which binds the active agent to the surface of the macromolecular nanoparticle structure by an adsorption process. This might render the nucleic acid material susceptible to the action of nucleases. Moreover, only small amounts of nucleic acid matter can be bound to the surface of the nanoparticles mentioned above. It is, therefore, desirable to provide an improved method for incorporating within nanoparticles larger amounts of macromolecules of the nucleic acid type, or, more generally, any RNA- and DNA-based active agents within nanoparticles.

The approach to this problem consists of the inventive proposal that the therapeutic agents are encapsulated or embedded within the cores of the nanoparticles to be administered. To effect this, a homogeneous organic phase is generated containing the therapeutic agent and the polymer from which the nanoparticles are formed. This phase is transferred to a separate aqueous phase partially miscible with the organic phase, thus forming an aqueous emulsion. Water is then added to induce the formation of an aqueous dispersion of the nanoparticles carrying the therapeutic agent.

### Summary of the Invention

The present invention relates to a pharmaceutical composition suitable for the incorporation of RNA- and DNA-based active agents in nanoparticles, characterized by the following components:
a) the therapeutic agent to be encapsulated or embedded within nanoparticles by dispersion methods;
b) a pharmaceutically acceptable biodegradable polymer which is suitable for the formation of nanoparticles;
c) and, optionally, further pharmaceutically acceptable additives which are suitable for incorporation in a dosage form for the intended mode of administration.

The pharmaceutical composition according to the present invention has distinct advantages, such as
- The nanoparticles are subject to endocytosis and facilitate the transport of active substances into cellular structures across the cytoplasmic membrane. This is a barrier which is usually impermeable to such substances. In particular, nanoparticles facilitate intracellular penetration of RNA- and DNA-based active agents by endocytosis and other pathways;
- The pharmacokinetic profile of the embedded therapeutic agent is advantageously modified. Nanoparticles may be considered as submicroscopic delivery units releasing the therapeutic agent in a controlled manner of release;
- Therapeutic agents which are sensitive to metabolism or degradation are "protected" by the polymeric structure of nanoparticles;
- Immunomodulating effects are obtained by stimulating immune reactions, which are elicited by the phagocytosis of the nanoparticles

These effects result in further advantages, such as the reduction of the amount of therapeutic agent required when using nanoparticles as compared to the amount required when using the free therapeutic agent, or the reduction of the necessary frequency of administration of the active substance.

### Detailed Description of the Invention

The general terms used throughout the specification of this invention are preferably defined as follows:

The term pharmaceutical composition defines a mixture containing the therapeutic agents, i.e. RNA- and DNA-based active agents, to be administered in a suitable dosage form to a host (humans or animals) in a therapeutic method of preventing or treating the disease or condition indicated. The preferred mode of administration is effected by parenteral, particularly the intravenous (i.v.), or intramuscular (i.m.) and in-situ administration, but also oral administration.

Nanoparticles and different methods for their preparation have been described in the scientific literature in numerous publications. They have a size from about 10 to 1000 nm. Considerable research work has been done to investigate the structure and therapeutic uses of nanoparticles.

A clear distinction is made between nanocapsules and nanospheres, cf. *E. Allémann et al., Eur. J. Pharmaceutics and Biopharmaceutics, **39** (1993), No.5, pages 173-191.* Nanocapsules have a polymeric shell and an inner liquid core. In this case, the therapeutic agent is usually dispersed in the inner core, but may also be adsorbed at the surface. Nanospheres have a matrix type structure. The therapeutic agent may be adsorbed at the surface, encapsulated, embedded or entrapped in the particle or dissolved in it. In a preferred embodiment of the present invention, RNA- and DNA-based active agents are incorporated within nanospheres.

Component a) - Therapeutic agent: The term RNA- and DNA-based active agents comprises in a non-limiting manner a composition of matter selected from the group consisting of oligonucleotides having 5 to 50 nucleotide units, oligonucleotide clamps of at least two oligonucleotide units, polynucleotides having more than 50 nucleotide units, nucleic acid fragments, genes and gene fragments.

As used in the context of the invention, the term oligonucleotide refers to a polymeric substance having a plurality of nucleotide units formed from naturally occurring bases and sugars, wherein the sugars are joined by phosphodiester internucleoside (backbone) linkages. The term oligonucleotide also includes analogues which consist at least partially of monomers (nucleotides) or portions thereof which do not occur naturally or which have linkages other than phosphodiester bonds. These oligonucleotide analogues are sometimes preferred over native forms because of properties such as enhanced cellular uptake, enhanced target binding affinity and increased stability in the presence of nucleases.

The term oligonucleotide also includes analogues having 5 to 50 nucleotide units specifically hybridizable with a DNA or RNA target. Hybridisation, in the context of nucleic acid chemistry, means hydrogen bonding, also known as Watson-Crick base pairing, between complementary bases, usually on opposite nucleic acid strands or two regions of a nucleic acid strand. Guanine and cytosine are examples of complementary bases which are known to form hydrogen bonds between them. "Specifically hybridizable" and "complementary" are terms which are used to indicate a sufficient degree of complementary such that stable and specific binding occurs between the DNA or RNA target and the oligonucleotide. It is understood that an oligonucleotide does not have to be 100% complementary to its target nucleic acid sequence to be specifically hybridizable.

An oligonucleotide is specifically hybridizable when binding of the oligonucleotide to the target interferes with the normal function of the target molecule, so as to cause a loss of biological functions, such as eliciting mRNA-synthesis or replication of the double or single stranded DNA or the reverse transcription. It is specifically hybridizable when there is a sufficient degree of complementarity to avoid non-specific binding of the oligonucleotide to non-target sequences under conditions in which specific binding is desired, i.e. under physiological conditions in the case of *in-vivo* assays or therapeutic treatment or, in the case of *in-vitro* assays, conditions under which the assays are conducted.

The relationship between an oligonucleotide and its complementary nucleic acid target to which it hybridizes is commonly referred to as "antisense". Targeting an oligonucleotide to a mRNA is commonly referred to as "antisense strategy", whereas targeting the oligonucleotide to a DNA is referred to as "antigene strategy", and may involve multistep procedures. The procedures usually begin with identifying a nucleic acid sequence whose function is to be modulated. This may be, as examples, a cellular gene (or mRNA made from the gene) whose expression is associated with a particular disease state, or a foreign nucleic acid of an infectious agent. The targeting procedure also includes determination of a site or sites within the nucleic acid sequence favoring the oligonucleotide interaction, which will result in the desired effect. Once the target sites have been identified, oligonucleotides are chosen which are sufficiently complementary to the target, i.e. hybridize sufficiently well and with sufficient specifity, so as to give the desired interference.

Preferred oligonucleotides are targeted to mRNA and to pre-mRNA. The term mRNA includes not only the coding region which carries the information to encode the protein using the three letter genetic code, but also associated ribonucleotides which form a region known as the 3'- or the 5'-untranslated region. The term pre-mRNA also includes intron regions and other sequences which are removed during mRNA maturation, the 5'-cap region, intron regions and intron/exon or splice junction ribonucleotides. Thus, oligonucleotides may be targeted wholly or in part to coding ribonucleotides as well as to associated ribonucleotides. The functions of mRNA and pre-mRNA to be interfered with include all biological functions such as translocation of the RNA to the site for protein translation, actual translation of protein from the mRNA, splicing or maturation of the pre-mRNA, and possibly even catalytic activity to be engaged in by the RNA. The present invention also relates to oligonucleotides directed towards coding and non-coding regions of mRNA.

Throughout this application, references to specific nucleotides are to nucleotides present on the coding strand of the nucleic acid. The following standard abbreviations are used throughout the specification to indicate specific nucleotides:
- C =: Cytidine
- T =: Thymidine
- A =: Adenosine
- G =: Guanosine

The oligonucleotide may be an oligodeoxyribonucleotide or an oligoribonucleotide (ribozymes) which can be linked by phosphodiester, phosphorothioate, phosphorodithoate or methylphosphonate bonds. The oligonucleotide sequence may be either in a regular or non-regular steric configuration.

A homopolymer is a sequence of repeating cytidine, thymidine, adenosine, or guanosine nucleotides or other natural bases thereof. For example, SdC28 is phosphorothioate oligonucleotide that is a homopolymer of cytidine of 28 base sequence length. The oligonucleotide may preferably consist of 5 to 50 nucleotides. Phosphorothioate may be either in a regular or non-regular steric configuration. The oligonucleotides may further be linked to a 3'- or 5'-cholesteryl moiety. Alternatively, the native phosphodiester may be modified in the bridge wherein the one of the two oxygen atoms involved in the bridge are replaced with bivalent groups such as -NH-, -CH₂-, or -S-. Phosphorothioate (PS) is an oligodeoxynucleotide in which the sulfur atom replaces one of the non-bridging oxygen atoms at each interbase phosphorus atom. The phosphorothioate can occur at each PS linkage either as Rp or Sp diastereomers.

Oligonucleotides may also be substituted or modified in the internucleotide phosphate residue with a thioether, carbamate, carbonate, acetamidate, or carboxymethyl ester group. In addition, the oligonucleotides may be substituted or modified in the sugar moiety with a ribose, 2'-allyl, glucose, sucrose, or galactose or any other sugar. Alternatively, the oligonucleotide may be substituted or modified in its 2'-position, such as 2'-O-methylribonucleotide. The oligonucleotide may also be substituted or modified to form an α-anomeric sugar. In addition, the oligonucleotide may be substituted or modified in its bases. Apart from the bases adenine, guanine, cytosine, and thymine, other natural bases such as inosine, deoxyinosine, or hypoxanthine are acceptable. In addition, isosteric purine 2'-deoxyxanthosine, or other purine or pyrimidine analogues may also be used. The oligonucleotide may be linked at the 5'-end with: intercalating agents, such as acridine derivatives; cross-linkers, such as psoralen-derivatives, azidophenacyl, proflavin, or azidoproflavin; artificial endonucleases which comprise those conjugates whose nuclease component is able, as such, to cleave DNA specifically and non-specifically, and acquires specificity by covalent linkage to the oligonucleotide, such as metal complexes EDTA-Fe(II), o-phenanthroine-Cu(I), and porphyrin-Fe(II); and lipophilic or peptide conjugates, such as long chain alcohols as phosphate esters, amino or mercapto groups, dyes or radioactive markers and polylysine. The oligonucleotide may be linked at the 3'-end with: intercalating agents, such as 2-methoxy-6-chloroacridine, methyl phosphonates, methyl esters, and aminoalkyls; alkylating oligonucleotides, such as amino-1-hexapolystaphylococcal nuclease; and alkaline phosphatase, terminal transferases, peptide conjugates, or polylysine. The oligonucleotides may be conjugated to a carbohydrate, sulfated carbohydrate, or glycan. Conjugates may be regarded in such a way as to introduce a specificity into otherwise unspecific DNA binding molecules by covalently linking them to a selectively hybridizing oligonucleotide.

The oligonucleotides present in the pharmaceutical composition may be prepared by using known methods such as solid phase synthesis. Equipment for such synthesis is available commercially from various sources including Applied Biosystems. The use of such methods to prepare oligonucleotides such as the phosphorothioates and alkylated derivatives is well known. It is also well known to use similar methods and commercially available modified amidites and controlled-pore glass (CPG) products such as biotin, fluorescein, acridine, or psoralen-modified amidites and/or CPG (available from Glen Research, Sterling VA (USA)) to synthesize fluorescently labeled, biotinylated or otherwise modified oligonucleotides such as cholesterol-modified oligonucleotides.

Oligonucleotide clamps of at least two oligonucleotide units can bind to a RNA sequence (the oligonucleotide units are bound by Watson/Crick pairs and they bind to the RNA sequence by Hoogsteen bonds). The oligonucleotide units can be free, linked together to form a clamp or bound as a circular molecule. These structures form triple helixes with RNA sequences. Polynucleotides, nucleic acid fragments, genes and gene fragments can be natural or synthetic; their size is above 100 nucleotides, they can be single- or double-stranded.

In a preferred embodiment of the invention, the RNA- and DNA-based active agents present in the pharmaceutical composition are selected from the group consisting of oligonucleotides having 5 to 50 nucleotide units, oligonucleotide clamps of at least two oligonucleotide units, polynucleotides, nucleic acid fragments, genes and gene fragments.

In another preferred embodiment of the invention, the oligonucleotide agents present in the pharmaceutical composition consist of single-stranded ribonucleic acids or deoxyribonucleic acid of natural or synthetic origin; bases of the acid(s) can be bound by phosphodiester bonds or bonds more resistant to nucleases selected from the group consisting of phosphonate, phosphotriester, phosphoramidate, phosphorothioate and methylphosphonate bonds.

In other preferred embodiments of the invention
- the oligonucleotide agents present in the pharmaceutical composition consists of single-stranded ribonucleic acids or deoxyribonucleic acid of natural or synthetic origin, the sugars of which can be bound by phosphodiester bonds or bonds more resistant to nucleases selected from the group consisting of phosphonate, phosphotriester, phosphoramidate, phosphorothioate and methylphosphonate bonds;
- the oligonucleotide consists of 5 to 50 nucleotides and is of natural or synthetic origin and may be substituted or modified in its phosphate, sugar or base moieties;
- the oligonucleotides are in the form of oligonucleotide clamps and can bind to a RNA-sequence, be free, linked together to form a clamp or bound as circular molecule and form triple helixes with RNA-sequences;
- the polynucleotides, nucleic acid fragments, genes and gene fragments are synthetic or of natural origin, single- or double-stranded and have a size above 100 bases;
- the oligonucleotides (the carrier) are attached by a linker to a "war head" and they recognize specific viral or cellular genes;
- the RNA- and DNA-based active agents are linked to a fluorescent dye;
- the RNA- and DNA-based active agents have hydrophilic properties, are modified or unmodified, bearing an overall positive or negative charge and are associated with a hydrophobic derivative having the opposite charge of the mentioned RNA- and DNA-based active agents;
- the RNA- and DNA-based active agents have hydrophilic properties and are associated with an uncharged hydrophobic derivative.

The term "dispersion methods" is used to distinguish the method of preparing the drug loaded nanoparticles, present in the pharmaceutical composition according to the present invention, from prior art methods of preparing drug loaded nanoparticles such as the one disclosed in *French Published Patent Application No. 2 724 935* mentioned above. The dispersion method is distinct from the adsorption method as the therapeutic agent is usually incorporated within the core of nanoparticles, preferably nanospheres. The process steps which define the dispersion method and which render the nanoparticles containing the encapsulated or embedded therapeutic agents distinct from loaded nanoparticles obtained by adsorption methods, are defined below.

The term "encapsulation", comprises various methods of incorporation, association or aggregation of the active therapeutic agents within the structures of the biodegradable polymers. The methods differ from the method of adsorption as disclosed in *French Published Patent Application No. 2 724 935* and comprises various known methods such as entrapment, embedding, or absorption.

Component b) - Polymers: A pharmaceutically acceptable biodegradable polymer, which is suitable for the formation of nanoparticles, is also suitable for use in the intended dosage form, particularly parenteral dosage forms such as intravenous or intramuscular injection formulations. Such a polymer is a pharmaceutically acceptable, biodegradable homopolymer or copolymer from monomers selected from the group consisting of L-lactide N or S, D-lactide S, D,L-lactide S, glycolide S and caprolactone. These polymers are marketed under the trade-mark MEDISORB (Registered Trade-Mark of Medisorb Technologies Inc.), PURASORB (Registered Trade-Mark of PURAC Biochem.) or RESOMER (Registered Trademark of Boehringer Ingelheim, Germany).
Suitable products are MEDISORB polymers from the L or DL series, e.g. 100 L or DL, or 8515, 7525, 6535, or 5050 DL, or RESOMER homopolymers from the L series, formed from L-lactide, e.g. L 104, 206 - 210, or 214, R series formed from racemic D,L-lactide, e.g. R 104, 202, 203, or 206 - 208 or G series formed from glycolide, e.g. G 205, or copolymers from the LR series formed from L-lactide with D,L-lactide, e.g. LR 708, or 909 or DL-lactide with glycolide, e.g. RG 502 - 505, 752, 755, 756, or 858.
In a preferred embodiment of the invention, the polymer suitable for the formation of nanoparticles is a pharmaceutically acceptable, biodegradable block copolymer of polyethyleneglycol with polymers from monomers selected from the group consisting of L-lactide N or S, D-lactide S, D,L-lactide S, glycolide S and caprolactone.
A pharmaceutically acceptable biodegradable polymer, which is suitable for the formation of nanoparticles, is also suitable for use in oral dosage forms containing defined amounts of the therapeutic agent, such as capsules or sachets, but also liquid dosage forms, such as droplets, suspensions, or emulsions. Such polymers are resistant to gastric juice and soluble or permeable by intestinal juices. They inhibit the release of the active agent under the strongly acidic conditions present in gastric fluids, but allow the controlled release (required by drug targeting) of the active agent from nanoparticles in pH-neutral or slightly basic juices present in the small intestine. The largest amount of active agent is released in the duodenum, but some release in the jejunum is also possible.
A suitable polymer, which is resistant to gastric juice and soluble in intestinal juices, is a copolymer formed from monomers selected from the group consisting of methacrylic acid, methacrylic acid esters, acrylic acid and acrylic acid esters. Those polymers are commercially available from Röhm Pharma GmbH, Weiterstadt, Germany, marketed under the trademark EUDRAGIT (Registered Trademark of Röhm Pharma GmbH).
An especially preferred polymer is the 1:1- up to 1:2-copolymer which is resistant to gastric juice and disintegrates in intestinal juices and which is formed from monomers selected from the group consisting of methacrylic acid and methacrylic acid lower alkyl esters, such as the 1:1- up to 1:2-copolymer from methacrylic acid and methyl methacrylate. The 1:1-copolymers are marketed in the EUDRAGIT L series. The corresponding 1:2-copolymers are marketed in the EUDRAGIT S series.
An especially preferred polymer is the 1:1-copolymer of methacrylic acid and acrylic acid ethyl ester. This polymer is marketed under the product name EUDRAGIT L 100-55. An alternative polymer suitable for the formation of nanoparticles is polyvinyl acetate phthalate (PVAP) or a pharmaceutically acceptable cellulose derivative selected from the group consisting of hydroxypropyl methyl cellulose acetate succinate (HPMCAS), hydroxypropyl methyl cellulose phthalate (HPMCP), cellulose acetate phthalate (CAP), and cellulose acetate trimellitate (CAT).
HPMCP is marketed as aqueous dispersion by Eastman Kodak Corp.. HPMCP 50 (USP/NF type 220824) and HPMCP 55 (USP/NF type 200731) are especially preferred.
CAP is marketed as aqueous dispersion under the trademark AQUATERIC (Registered Trademark of FMC Corp.) or is commercially available from Eastman (composition: phthalyl 35 %, acetyl 24 %, moisture 1 %, free acid 0.5 % (as phthalic acid)).

CAT is commercially available from Eastman (composition: trimellityl 29 %, acetyl 22 %, moisture 1 %, free acid 0.5 % (as phthalic acid)).

Component c) - Additives: Pharmaceutically acceptable additives in addition to component b) are defined by the therapeutic requirements of the disease to be treated and the dosage form for the intended mode of administration. Such additives may support the desired mechanism of action, e.g. endosomal escape, by using suitable additives, e.g. hemaglutinin, which fuse endosomal membranes and facilitate the migration of the oligonucleotide into the cell.
A preferred mode of administration is i.v., but also i.m..If the intravenous administration is intended, water is added that has been sterilized and made free of pyrogens, according to the prescriptions of national pharmacopoeias, such as U.S. Pharmacopoeia (USP) or Deutsches Arzneibuch (DAB). The addition of water-soluble additives, which are suitable for the adjustment of isotonic conditions, is particularly preferred. Such additives may be sodium chloride, sorbitan, mannitol, glucose, trehalose, lactose or fructose. If the intramuscular administration is intended, oily carrier liquids, such as sesame oil or olive oil, but also lecithin, are particularly preferred.
Oral dosage forms are in particular solid oral dosage forms containing defined amounts of the active agent, such as capsules or sachets, but also liquid dosage forms, such as droplets, suspensions, or emulsions. Capsules are dry-filled capsules made of gelatin, especially hard gelatin, which are prepared, where appropriate, with the addition of solid excipients, and which are dissolved without time delay by the action of gastric juice to release the components a) and b). Suitable excipients such as sorbitol, lactose, starch, or magnesium stearate, may be admixed. Soft capsules may contain the liquid dosage forms mentioned, in particular suspensions or emulsions. They may contain, as additives, glycerol, lecithin, fats, oil, paraffin oil or liquid polyethylene glycol. Dry-filled capsule sizes 0-4, preferably 0-2, are suitable, depending on the dose to be administered. Commercial products marketed by Eli Lilly, Elanco, Capsugel, Shionogi, or Scherer are suitable.

Method of Preparation. The invention also relates to a process for the preparation of the pharmaceutical composition defined above which is characterized in that an aqueous dispersion of nanoparticles is formed containing a) RNA- and DNA-based active agents to be incorporated; and b) the pharmaceutically acceptable hydrophilic polymer, which is suitable for the formation of an aqueous dispersion of nanoparticles; and the dispersion is processed further under the optional addition of pharmaceutically acceptable additives c) which are suitable for the incorporation in the dosage form for the intended mode of administration. Various methods for performing this process are known. They are compiled in the publication of *Eric Allémann et al., loc. cit..*The methods for the preparation of nanospheres mentioned in this reference are particulary preferred.
An especially preferred method comprises the preparation of an aqueous gel containing a hydrophilic polymer under the optional addition of a water soluble salt. This gel is then mixed with a solution of an organic solvent containing the therapeutic agent and the polymer which is suitable for the formation of nanoparticles. Emulsification is then observed, and after addition of water the nanoparticles are formed and homogeneously dispersed in the aqueous phase. The aqueous phase is then processed further to the pharmaceutical dosage form intended, e.g. by applying conventional purification and separation methods. The preparation of the aqueous gel containing the hydrophilic polymer is disclosed in the reference of E. Allémann, loc. cit. and the references cited therein. The gel is formed by the addition of water to the hydrophilic polymer. Suitable hydrophilic polymers are polyvinyl alcohols, such as the ones marketed under the trademark MOWIOL (Registered Trademark of Hoechst AG, Germany). Preferred are polyvinyl alcohols having a degree of hydrolysis of more than 70 % (partially hydrolyzed grades), especially more than 87 %, e.g. MOWIOL from the 88 and 92 series, e.g. 4-88, 5-88, 8-88, 18-88, 23-88, 26-88, and 40-88. To facilitate the phase separation from the organic phase, which is subsequently added, the addition of a physiologically acceptable water-soluble salt, e.g. magnesium chloride, or magnesium acetate, to the gel phase is suggested.
The gel phase is added under stirring to a solution of the organic solvent, e.g. acetone or benzyl alcohol, which contains the therapeutic agent, and the pharmaceutically acceptable biodegradable polymer which is suitable for the formation of nanoparticles defined above, especially biodegradable block copolymers of polyethyleneglycol with polymers from monomers selected from the group consisting of L-lactide N or S, D-lactide S, D,L-lactide S, glycolide S and caprolactone.
In a preferred embodiment of the process, RNA- and DNA-based active agents, especially oligonucleotides, are dispersed in water under the addition of pharmaceutically acceptable additives which render the active agents more lipophilic, such as surfactants, particularly cationic surfactants of the cetyltrimethylammonium type (CTAB), centrifuged, separated from the supernatant aqueous phase and dried. The dried residue is then added to the organic solvent.

Distilled water is then added to the emulsion to allow the diffusion of the organic solvent into the aqueous phase, and the nanoparticles are formed and homogeneously dispersed therein. The organic solvent and undesired preparation additives can be eliminated by purification methods, such as ultracentrifugation or cross flow filtration.
The homogeneous dispersion obtained may be defined as an aqueous suspension of nanoparticles containing the therapeutic agent, e.g. RNA- and DNA-based active agents mentioned above. According to the preferred method of phase separation of the aqueous gel from the organic solvent, a homogeneous dispersion of nanospheres is obtained. Nanospheres are distinguishable from nanocapsules, cf. *E. Allémann et al., loc. cit.,* especially with physical methods, such as photon correlation spectroscopy (PCS), e. g. with a COULTER NANO-SIZER, LASER light scattering methods, or electron microscopy. Nanospheres are also distinguishable from other carrier particles, such as liquid crystals, micells, reversed micells, liposomes, microspheres or microcapsules. For a statistical average of more than 80 %, preferably more than 90 %, a mean average particle size between 60 and 1000 nm has been determined. The size of the nanoparticles obtained depends on the established and known methods chosen for their preparation.
The homogeneous aqueous dispersion containing nanospheres is then processed further to a conventional pharmaceutical dosage form by applying standard purification methods, such as the ones known in the art for purifying nanoparticles, such as ultracentrifugation, cross-flow filtration, or sterile filtration. The dispersion can also be lyophilized in a conventional manner, and the lyophilisate is then reconstituted to the pharmaceutical dosage form desired.
The homogeneous aqueous dispersion, optionally after concentration to standardized volumes, or the lyophilisate is added to suitable containers for unitary dosage forms, such as vials.
The homogeneous dispersion containing the nanospheres may also be converted to a lyophilisate which is then reconstituted by the addition of water before administerin the dispersion, e.g. parenterally. Even after reconstituting the lyophilisate, a homogeneous nanodispersion is formed again. When preparing lyophilisates, the addition of calculated amounts of water soluble additives is recommended to obtain isotonic conditions after parenteral administration of the reconstituted dispersion.

Uses. The pharmaceutical compositions according to the invention may be administered by pulmonary delivery or, preferably, parenterally, for example intravenously, subcutaneously, intraperitoneally or intramuscularly. The dosage depends principally on the method of administration and on the severity and responsiveness of the condition to be treated. Individual doses and the administration regime can best be determined by individual judgement of a particular case of illness. Diseases which may be treated with the composition include human and animal cancers, such as lung cancer, stomach cancer, renal cancer, breast cancer, laryngeal cancer, pancreatic cancer, colorectal cancer, prostate cancer, malignant melanoma, and viral diseases, such as AIDS. Treatment also includes preventive interference (e. g. in the case of human papilloma virus infection capable of causing cervix carcinoma after a latency period).
The nanoparticles present in the compositions are also useful as so-called drug carriers for oligonucleotides and an almost unlimited range of applications such as antisense strategy (binding of oligonucleotides to mRNA or to viral RNA), binding and cleavage of RNA (ribozyme action), anti-gene strategy (triple helix formation with DNA and inhibition of gene expression), aptamer strategy (the binding to target molecules such as enzymes and proteins, e.g. binding to thrombin). The nanoparticles may also be used as drug carriers for oligonucleotides carrying active principles of the type "smart biological bombs" according to the method of Watanabe K. A. et al., U.S.Patent No. 5,571,937. These oligonucleotides are programmed to recognize and to bind specific viral or cellular genes and are attached by a linker to a "warhead" capable of destroying, inactivating, or blocking the said genes. The "warheads" can be different types of organic molecules which, upon interaction with the target DNA sequence, inactivate the gene either by double-strand cleavage or by forming a covalent bond. Primarily the "bombs" are intended specifically for the inactivation of intracellular HIV DNA molecules or human papilloma viral DNA molecules. Analogous "bombs" can also be programmed to interact with and inactivate other human or animal DNA viruses and RNA viruses which by reverse transcription reverse their RNA genome into DNA. Nanoparticles are also useful to detect precancerous lesions deep within tissues or organs.
Further applications and uses of the compositions as drug carriers for gene constructs as gene markers, for DNA constructs in the production of active RNA oligonucleotides within the cells, or for genes in gene replacement or therapy in the transfection of cells with non-resident genes in order to accomplish in-situ expression of therapeutic agents such as proteins, immunotoxins, hormones or enzymes, are also within the scope of the present invention. The invention is illustrated by the Examples:

### Example 1

### a) Production of an oligonucleotide hydrophobic derivative.

The oligonucleotide derivative is a complex composed of the oligonucleotide SdT15 which is prepared according to the method as described in *Stein C. et al., Biochemistry, 1993, **32**, 4855-4861* and a cationic detergent, cetyltrimethylammonium bromide (CTAB). The complex is formed as a result of ionic bond formation between the nitrogen cations of CTAB and the anionic phosphate groups of the oligo. The resulting complex is insoluble in water.

A 7.5 % (m/v) CTAB (Fluka Chemicals) is prepared in distilled water. 1.5 µl of CTAB solution is added to 1624 µg of oligonucleotide in a volume of 58 µl distilled water and mixed gently. The mixture is centrifuged for 3 minutes at a speed of 12 000 rpm. Without disruption of the pellet residue, 0.5 µl of CTAB solution is added to the supernatant and the mixture is again centrifuged for 3 minutes at a speed of 12 000 rpm. Further aliquots of CTAB solution are added and the sample centrifuged until no cloudiness is seen in the supernatant. The sample is then centrifuged for 15 minutes at 12 000 rpm and the supernatant discarded. The pellet residue containing the oligo-CTAB complex is then dried under vacuum.

### b) Production of a nanoparticle dispersion (200 - 300 nm) loaded with a hydrophobic oligo derivative.

Organic Phase: A solution of 800 µg of the dried oligo derivative in 40 µl benzyl alcohol (Fluka Chemicals) is added to 1.06 ml of a solution containing 5 % PLA [poly(D,L lactic acid, Medisorb] in benzyl alcohol. Aqueous Phase: A solution of 15 % polyvinyl alcohol (Mowiol® 4-88, Hoechst Germany) is prepared in distilled water.

1.1 ml of the organic phase are weighed into a glass reaction vessel (internal diameter 29 mm, length 100 mm). 2 ml of the aqueous phase are then added to the organic phase using a 5 ml syringe over a period of 2.5 min., whilst the mixture is being stirred at a speed of 1400 rpm using a homogenizer with a 15 mm diameter paddle attachment. 33 ml of distilled water is then added to the emulsion, using a 50 ml syringe, over a period of 4.5 minutes whilst the mixture is being stirred at a speed of 1400 rpm. After the water has been added, the mixture is allowed to continue stirring at the same speed for 10 minutes. The nanoparticle suspension is then divided into 3 ml aliquot parts and centrifuged at 15 000 g for 20 minutes in a Beckman Avanti 30 centrifuge. The clear supernatant is discarded and the nanoparticle pellet residue is resuspended in approximately 4 ml of distilled water by aspiration using a glass Pasteur pipette. The samples are then centrifuged at 15 000 g for 15 minutes and the previous step is repeated. The samples are again centrifuged at 15 000 g for 10 minutes and the nanoparticle pellet residues are resuspended in 1 ml of distilled water and pooled to give a total volume of 6 ml. The pooled samples are then freeze-dried for 2 days using a LSL Secfroid lyolab BII. The lyophilisate is stored in the dark in a freezer.

### c) Conversion to an intravenous dosage form

The freeze dried nanoparticles are resuspended in isotonic sodium chloride solution by vortexing.

### d) Characterization of oligonucleotide loaded PLA nanoparticles

The size of the nanoparticles is assessed by photon correlation spectroscopy using a Coulter Nanosizer. The morphology of the nanoparticles is determined by scanning electron microscopy. The therapeutic agent content is assessed by spectrophotometry after dissolution of the freeze dried nanoparticles in an appropriate solvent such as chloroform. After extraction in a sodium hydroxide solution, the amount of oligonucleotide is then measured using UV absorbance at 270 nm.

### Example 2

### Production of a nanoparticle dispersion (800 - 900 nm)

In a manner analogous to Example 1, a nanoparticle dispersion (800 - 900 nm) is prepared by mixing the organic phase with the aqueous phase containing 10 % polyvinyl alcohol (Mowiol® 4-88). All other process steps are analogous.

### Example 3

### Production of a nanoparticle dispersion containing other oligonucleotides

In a manner analogous to Example 1, a nanoparticle dispersion is prepared containing the following oligonucleotides: SdC28 or SdC15.

## Claims

1. A pharmaceutical composition suitable for the incorporation of RNA- and DNA-based active agents in nanoparticles, characterized by the following components:
a) the active agent to be encapsulated or embedded within nanoparticles by dispersion methods;
b) a pharmaceutically acceptable biodegradable polymer which is suitable for the formation of nanoparticles;
c) and, optionally, further pharmaceutically acceptable additives which are suitable for the incorporation in a dosage form for the intended mode of administration.

2. A pharmaceutical composition according to claim 1, wherein the RNA- and DNA-based active agents are selected from the group consisting of oligonucleotides having 5 to 50 nucleotide units, oligonucleotide clamps of at least two oligonucleotide units, polynucleotides, nucleic acid fragments, genes and gene fragments.

3. A pharmaceutical composition according to claim 2, wherein the oligonucleotide consists of single-stranded ribonucleic acids or deoxyribonucleic acid of natural or synthetic origin, the bases of which can be bound by phosphodiester bonds or bonds more resistant to nucleases selected from the group consisting of phosphonate, phosphotriester, phosphoramidate, phosphorothioate and methylphosphonate bonds.

4. A pharmaceutical composition according to claim 2, wherein the oligonucleotide consists of 5 to 50 nucleotide units and is of natural or synthetic origin and may be substituted or modified in its phosphate, sugar or base moieties.

5. A pharmaceutical composition according to claim 2, wherein the oligonucleotides in the oligonucleotide clamps can bind to a RNA-sequence, be free, linked together to form a clamp or bound as circular molecule and form triple helixes with RNA-sequences.

6. A pharmaceutical composition according to claim 2, wherein the polynucleotides, nucleic acid fragments, genes and gene fragments are synthetic or of natural origin, single- or double- stranded and have a size above 100 bases.

7. A pharmaceutical composition according to claim 2, wherein the oligonucleotides are programmed to recognize and to bind specific viral or cellular genes and are attached by a linker to a "warhead" capable of destroying, inactivating, or blocking the said genes.

8. A pharmaceutical composition according to claim 1, wherein the RNA- and DNA-based active agents are linked to a fluorescent dye.

9. A pharmaceutical composition according to claim 1, wherein the RNA- and DNA-based active agents have hydrophilic properties, are modified or unmodified, bearing an overall positive or negative charge and are associated with a hydrophobic derivative having the opposite charge of the mentioned RNA- and DNA-based active agents.

10. A pharmaceutical composition according to claim 1, wherein the RNA- and DNA-based active agents have hydrophilic properties and are associated with an uncharged hydrophobic derivative.

11. A pharmaceutical composition according to claim 1, wherein the polymer suitable for the formation of nanoparticles is a pharmaceutically acceptable, biodegradable homopolymer or copolymer from monomers selected from the group consisting of L-lactide N or S, D-lactide S, D,L-lactide S, glycolide S and caprolactone.

12. A pharmaceutical composition according to claim 1, wherein the polymer suitable for the formation of nanoparticles is a pharmaceutically acceptable, biodegradable block copolymer of polyethyleneglycol with polymers from monomers selected from the group consisting of L-lactide N or S, D-lactide S, D,L-lactide S, glycolide S and caprolactone.

13. A pharmaceutical composition according to claim 1, wherein pharmaceutically acceptable additives are selected which are suitable for the incorporation in a dosage form for parenteral or oral administration.

14. A pharmaceutical composition according to claim 1, wherein the polymer suitable for the formation of nanoparticles is a 1:1- up to 1:2-copolymer which is resistant to gastric juice and soluble or disintegrating in intestinal juices and which is formed from monomers selected from the group consisting of methacrylic acid and methacrylic acid lower alkyl esters, polyvinyl acetate phthtalate (PVAP) or a pharmaceutically acceptable cellulose succinate (HPMCAS), hydroxypropyl methyl cellulose phthalate (HPMCP), cellulose acetate phthalate (CAP), and cellulose acetate trimellitate (CAT).

15. A process for the preparation of the pharmaceutical composition according to claim 1,characterized in that an aqueous dispersion of nanoparticles is formed containing a) RNA- and DNA-based active agents to beincorporated; and b) the pharmaceutically acceptable polymer, which is suitable for the formation of an aqueous dispersion of nanoparticles; and the dispersion is processed further under the optional addition of pharmaceutically acceptable additives c), which are suitable for the incorporation in the dosage form for the intended mode of administration.

16. A process according to claim 14, characterized in that the aqueous dispersion of nanospheres is processed further to a lyophilisate.

17. A pharmaceutical composition according to claim 1 for use in a method for treating the human or animal body by therapy.
